(19) Europäisches Patentamt
European Patent Office
Office européen des brevets

(11) **EP 4 100 780 B1**

(12) **EUROPEAN PATENT SPECIFICATION**

(45) Date of publication and mention
of the grant of the patent:
**20.11.2024 Bulletin 2024/47**

(21) Application number: **21702691.3**

(22) Date of filing: **08.02.2021**

(51) International Patent Classification (IPC):
**G02B 27/00** (2006.01)   **A61B 3/00** (2006.01)
**A61B 3/02** (2006.01)   **A61B 3/028** (2006.01)
**A61B 3/036** (2006.01)   **G02B 27/01** (2006.01)
**A61B 3/032** (2006.01)

(52) Cooperative Patent Classification (CPC):
**G02B 27/017; A61B 3/0033; A61B 3/005;
A61B 3/032; G02B 27/0093;** A61B 3/036

(86) International application number:
**PCT/EP2021/052926**

(87) International publication number:
**WO 2021/156503 (12.08.2021 Gazette 2021/32)**

(54) **METHOD FOR DISPLAYING A SHARP IMAGE ON A RETINA OF AN EYE OF THE PERSON**

VERFAHREN ZUR ANZEIGE EINES SCHARFEN BILDES AUF EINER NETZHAUT EINES AUGES EINER PERSON

PROCÉDÉ D'AFFICHAGE D'UNE IMAGE NETTE SUR LA RÉTINE D'UN IL DE LA PERSONNE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **07.02.2020 EP 20305116**

(43) Date of publication of application:
**14.12.2022 Bulletin 2022/50**

(73) Proprietor: **Essilor International
94220 Charenton-Le-Pont (FR)**

(72) Inventors:
• **PELOUX, Marius
94220 Charenton-Le-Pont (FR)**
• **BOUTINON, Stéphane
94220 Charenton-Le-Pont (FR)**
• **ROPTIN, Vincent
94220 Charenton-Le-Pont (FR)**

(74) Representative: **Cabinet Novitech
188 Grande rue Charles de Gaulle
94130 Nogent-sur-Marne (FR)**

(56) References cited:
**EP-A1- 3 296 797       EP-B1- 3 296 797
US-A1- 2008 117 289    US-A1- 2016 026 253
US-A1- 2017 000 335    US-B2- 10 444 507
US-B2- 10 444 508      US-B2- 10 451 895**

• **FITZKE F W ET AL: "A maxwellian-view
optometer suitable for electrophysiological and
psychophysical research", VISION RESEARCH,
ELSEVIER, AMSTERDAM, NL, vol. 25, no. 6, 1
January 1985 (1985-01-01), pages 871 - 874,
XP024311297, ISSN: 0042-6989, [retrieved on
19850101], DOI: 10.1016/0042-6989(85)90196-8**

**Description**

FIELD OF THE INVENTION

The invention relates to a method for displaying a sharp image on the retina of an eye of a person. The invention further relates to a method for determining at least one parameter of an eye of a person.

BACKGROUND OF THE INVENTION

A head-mounted system (HMD) is an electro-optical device worn on the head by a wearer. Usually such system is electronically controlled so as to switch between different stages or to display information to the wearer. A head mounted system usually presents like a spectacle frame with electronically controlled spectacle lenses.

Head-mounted system are used according to various usage pattern such as non-immersive head-mounted system that allows the wearer to interact with their environment while using the head-mounted system or immersive head-mounted system that cuts off the field of outside view.

There is a need to provide a head mounted optical device, for example a head mounted display system, adapted to a wearer, in particular adapted to its visual needs in a simply manner and preferably in a late step of the manufacturing of the optical device in order to limit the unit production cost.

Indeed, if the wearer needs corrective ophthalmic lenses to see the real world correctly or the displayed information, the head mounted optical system should be adapted to such requirements.

Therefore, there is a need to provide an optical device, for example a head-mounted see-through system, adapted to a wearer and in particular to a wearer's prescription.

US 10 451 895 B2 discloses methods and a wearable ophthalmic device that may include an outward facing head-mounted light field camera to receive light from a user's surroundings and to generate numerical light field image data.

EP 3 296 797 A1 relates to methods and apparatus to display an image on a person's vision using a display built into eyewear.

SUMMARY OF THE INVENTION

To this end, the invention proposes a method for displaying a sharp image on a retina of an eye of the person, the person having a prescription for the eye of the person, the method comprising:

- providing at least one optical parameter relative to the prescription for the eye of the person;
- providing a plurality of initial sub-images, each initial sub-image corresponding to at least a part of the image to be displayed;
- providing a plurality of light beams configured to be focused substantially in the plane of a pupil of the eye at a plurality of corresponding different positions, each light beam being configured to carry an associated sub-image;
- for each sub-image, adapting the sub-image based on the at least one provided optical parameter and on the corresponding focused position of the light beam configured to carry the sub-image to form an adapted sub-image; and
- displaying each adapted sub-image carried by the associated light beam on the retina of the person.

Advantageously, such method allows customizing a head-mounted display device to the user's viewing ability. Indeed, such method allows a pre-compensation of the image to be displayed on the retina of the user based on the prescription of the eye of the user, so as to display a sharp image on the retina of the user. Thus, the use of the head-mounted display device and the virtual comfort of the virtual image is optimized for the user.

Consequently, a single head-mounted device could be used by different persons having different prescription, the image to be displayed on the retina of each person being corrected based on a prescription of the eye of the person determined by the same head-mounted device.

According to further embodiments which can be considered alone or in combination:

- adapting the sub-image comprises adapting the relative position of the sub-image in the image to be displayed;
- adapting the relative position of the sub-image in the image to be displayed comprises:

    • adapting a horizontal angular position of the sub-image carried by the associated light beam based on the at least one provided optical parameter and on the corresponding focused position of the associated light beam; and
    • adapting a vertical angular position of the sub-image carried by the associated light beam based on the at least one provided optical parameter and on the corresponding focused position of the associated light beam.

- the method further comprises for each light beam of the plurality of light beams determining the focused position in the plane of the pupil of the eye;
- the focused positions of the plurality of light beams are regularly distant from each other in the plane of the pupil of the eye;
- the adapted sub-images are displayed sequentially on the retina of the person;
- the adapted sub-images are displayed simultaneously on the retina of the person;
- a wavelength of at least one of the plurality of light beams differs from a wavelength of at least one other of the plurality of light beams;
- the wavelengths of the plurality of light beams are comprises in a narrow band of wavelengths of 50 nm width;
- at least three optical parameters relative to the prescription for the eye of the person are provided;
- the at least one provided optical parameters of the person's eye is relative to a dioptric power, an astigmatism and an axis of the eye of the person;
- providing at least one optical parameter relative to the prescription for the eye of the person comprises:

  • displaying at least two sharp images on a retina of the eye of the person, the at least two images comprising a target and being carried by two light beams focused substantially in the plane of a pupil of the eye at at least two different positions;
  • adapting a parameter of the target in each image based on feedback of the person relative to the change of the parameter of the target in the image; and
  • determining the at least one optical parameter of the person's eye based on the adaption of the parameter of the target in each image.

- the target is a spot;
- the target is a one-dimensional image;
- the target is a two-dimensional image.

According to a further aspect, the invention further relates to a display device adapted to provide a plurality of light beams configured to be focused substantially in the plane of a pupil of the eye at a plurality of corresponding different positions, each light beam being configured to carry an associated sub-image and adapted to execute at least the steps of the method of the invention.

According to a further aspect, the invention further relates to a device comprising a processor adapted to store one or more sequence of instructions and to carry out at least one of the steps of the method for displaying a sharp image on a retina of an eye of the person according to the invention.

More particularly, the invention relates to a computer program product comprising one or more stored sequences of instructions that are accessible to a processor and which, when executed by the processor, causes the processor to carry out at least the following steps of the method for determining at least one optical parameter of an eye of a person according to the invention, using a display device according to the invention:

- providing at least one optical parameter relative to the prescription for the eye of the person;
- providing a plurality of initial sub-images, each initial sub-image corresponding to at least a part of the image to be displayed;
- providing a plurality of light beams configured to be focused substantially in the plane of a pupil of the eye at a plurality of corresponding different positions, each light beam being configured to carry an associated sub-image;
- for each sub-image, adapting the sub-image based on the at least one provided optical parameter and on the corresponding focused position of the light beam configured to carry the sub-image to form an adapted sub-image; and
- displaying each adapted sub-image carried by the associated light beam on the retina of the person.

The invention further relates to a computer readable medium carrying one or more sequences of instructions of the computer program product according to the invention.

Unless specifically stated otherwise, as apparent from the following discussions, it is appreciated that throughout the specification discussions utilizing terms such as "computing", "calculating", or the like, refer to the action and/or processes of a computer or computing system, or similar electronic computing device, that manipulate and/or transform data represented as physical, such as electronic, quantities within the computing system's registers and/or memories into other data similarly represented as physical quantities within the computing system's memories, registers or other such information storage, transmission or display devices.

Embodiments of the present invention may include apparatuses for performing the operations herein. This apparatus may be specially constructed for the desired purposes, or it may comprise a general purpose computer or Digital Signal Processor ("DSP") selectively activated or reconfigured by a computer program stored in the computer. Such a computer program may be stored in a computer readable storage medium, such as, but is not limited to, any type of disk including floppy disks, optical disks, CD-ROMs, magnetic-optical disks, read-only memories (ROMs), random access memories (RAMs) electrically programmable read-only memories (EPROMs), electrically erasable and programmable read only memories (EEPROMs), magnetic or optical cards, or any other type of media suitable for storing electronic instructions, and capable of being coupled to a computer system bus.

The processes and displays presented herein are not inherently related to any particular computer or other apparatus. Various general purpose systems may be used with programs in accordance with the teachings herein, or it may prove convenient to construct a more specialized apparatus to perform the desired method. The desired structure for a variety of these systems will appear from the description below. In addition, embodiments of the present invention are not described with reference to any particular programming language. It will be appreciated that a variety of programming languages may be used to implement the teachings of the inventions as described herein.

BRIEF DESCRIPTION OF THE DRAWINGS

Embodiments of the invention will now be described, by way of example only, and with reference to the following drawings in which:

- Figure 1 is a diagram of a lens/eye optical system, seen from the side;
- Figures 2 and 3 are perspective diagrams of a lens/eye system;
- Figure 4 is an illustration of a chart-flow of a method for displaying a sharp image on a retina of an eye of a person according to the invention;
- Figure 5 illustrates resulting images as seen by a person having different visual defects;
- Figures 6 and 7 illustrate an image formation using a picoprojector respectively without and with a shift between the eye pupil and the optical axis of the picoprojector for an emmetropic eye;
- Figures 8 and 9 illustrate an image formation using a picoprojector respectively without and with a shift between the eye pupil and the optical axis of the picoprojector for an myopic eye;
- Figure 10 is an illustration of a schematic eye model;
- Figure 11 illustrates an image formation using two picoprojectors respectively according to a method for displaying a sharp image on a retina of an eye of an ametropic person, for example a myopic person; and
- Figures 12 and 13 are illustrations of chart-flows of a method for determining optical parameters of an eye of a person according to two embodiments of the invention.

Elements in the figures are illustrated for simplicity and clarity and have not necessarily been drawn to scale. For example, the dimensions of some of the elements in the figure may be exaggerated relative to other elements to help improve the understanding of the embodiments of the present invention.

DETAILED DESCRIPTION OF PREFERRED EMBODIMENTS

The present invention relates to a method displaying a sharp image on a retina of an eye of the person, the person having a prescription for the eye of the person.

As well-known, the ophthalmic prescription can include a positive or negative power prescription as well as an astigmatism prescription. These prescriptions correspond to corrections enabling the wearer of lenses to correct defects of his/her vision.

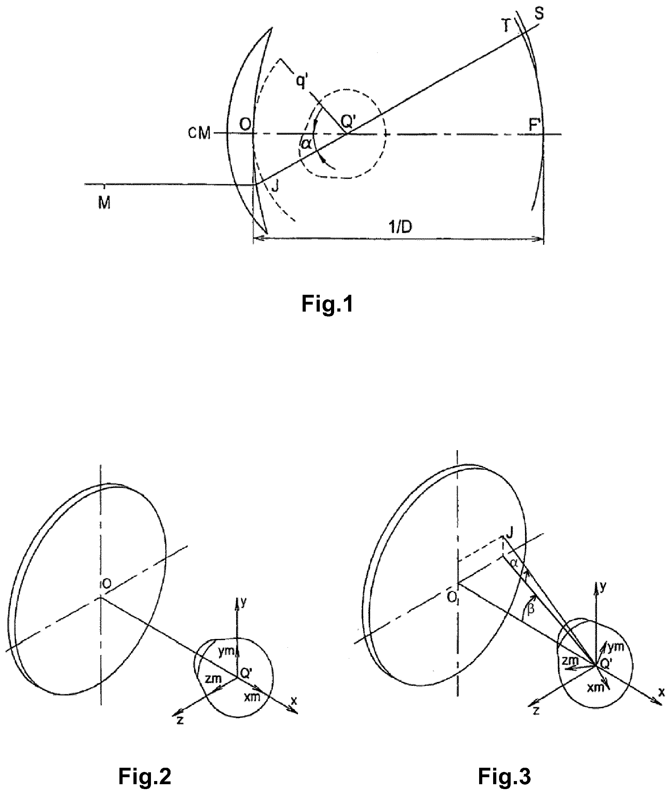

Conventionally, optical quantities, namely power and astigmatism, are defined for a given lens under the conditions in which it is to be worn. Figure 1 shows a diagram of a lens/eye optical system seen from the side, and shows the definitions used in the rest of the description based on an example of a progressive multifocal ophthalmic lens having a front complex surface.

The center of rotation of the eye is called Q'. The axis Q'F' shown in the figure by the dot/dash line is the horizontal axis passing through the center of rotation of the eye Q' and extending in front of the wearer; in other words the axis Q'F' corresponds to the primary viewing direction. This axis intersects the front face of the lens at a point called the fitting cross CM. The fitting cross is marked on lenses in order to allow them to be positioned by an optician. The fitting cross is generally located 4 mm above the geometric center of the front face of the lens.

An apex sphere also called vertex sphere, with center Q' and radius q', is defined, as the sphere that cuts the rear face of the lens at the point O corresponding to the intersection by the axis Q'F' of the rear face of the lens.

As an example, a value of the radius q' of 25.5 mm corresponds to a standard value and provides satisfactory results when the lenses are worn.

A given viewing direction-shown by the solid line in FIG. 1, corresponds to a position of the eye rotating about Q' and to a point J on the apex sphere.

A viewing direction may also be identified, in spherical coordinates, by two angles α and β, in the so called Fick system.

The angle α is the angle between the Q'F' axis and the projection of the straight line Q'J on the vertical plane containing the Q'F' axis, this angle appearing in the diagram of figure 1.

The angle β is the angle between the Q'F' axis and the projection of the straight line Q'J on the horizontal plane containing the Q'F' axis. A given viewing direction therefore corresponds to a point J on the apex sphere or to a coordinate pair (α,β).

In a given viewing direction, the image of a point M in the object space, located at a given object distance, is formed between two points S and T corresponding to minimum and maximum distances JS and JT (which would be the sagittal and tangential focal lengths in the case of surfaces of revolution and of a point M at infinity).

In the example of Figure 1, the image of a point in the object space at infinity is formed, on the Q'F' axis, at the point F'. The points S and T are coincident, which amounts to stating that the lens is locally spherical in the primary viewing direction. The distance D is the rear frontal plane of the lens.

Figures 2 and 3 show perspective diagrams of a lens/eye system.

Figure 2 shows the position of the eye and the reference frame associated with the eye, in the principal viewing direction, α=β=0, called the primary viewing direction. The points J and O are then coincident.

Figure 3 shows the position of the eye and the reference frame that is associated therewith in a direction (α,β).

Shown in figures 2 and 3 are a fixed reference frame {x,y,z} and a reference frame {xm,ym,zm} associated with the eye in order to show clearly the rotation of the eye. The reference frame {x,y,z} has as origin the point Q' and the x-axis is the Q'F' axis-the point F' not being shown in FIGS. 2 and 3 and passes through the point O. This axis is directed from the lens to the eye in correspondence with the direction of measurement of the astigmatism axis. The {y,z} plane is the vertical plane. The y-axis is vertical and directed upwards. The z-axis is horizontal, the reference frame being a direct orthonormal coordinate system. The reference frame {xm,ym,zm} associated with the eye has the point Q' as center. The xm axis is defined by the viewing direction JQ', and coincides with the {x,y,z} reference frame in the case of the primary viewing direction. Listing's law gives the relationships between the {x,y,z} and {xm,ym,zm} coordinate systems for each viewing direction-see Le Grand, Optique Physiologique, Volume 1, published by Revue d'Optique, Paris 1965.

The section of the lens may be drawn in the (O,x,y) plane defined with reference to figure 2. The tangent to this curve at the point O is inclined to the (O,y) axis at an angle called the pantoscopic angle.

It is also possible to draw the cut of the lens in the (O,x,z) plane. The tangent to this curve at the point O is inclined to the (O,z) axis at what is called the wrap angle.

Using these elements, it is possible to define a wearer optical power and astigmatism under usual wearing conditions, in each viewing direction.

An object point M at an object distance given by the ergorama is considered for a gaze direction (α,β).

The ergorama is a function associating to each gaze direction the usual distance of an object point. Typically, in far vision following the primary gaze direction, the object point is at infinity. In near vision, following a gaze direction essentially corresponding to an angle α of the order of 36.6° and to an angle β of the order of 6° in absolute value towards the nasal side, the object distance is of the order of 30 to 50 cm. For more details concerning a possible definition of an ergorama, US patent US-A-6,318,859 may be considered. This document describes an ergorama, its definition and its modeling method. For a method of the invention, points may be at infinity or not. Ergorama may be a function of the wearer's ametropia.

An object proximity ProxO is defined for the point M on the corresponding light ray in the object space as the inverse of the distance MJ between point M and point J of the apex sphere:

$$ProxO = \frac{1}{MJ}$$

This enables to calculate the object proximity within a thin lens approximation for all points of the apex sphere, which is used for the determination of the ergorama. For a real lens, the object proximity can be considered as the inverse of the distance between the object point and the front surface of the lens, on the corresponding light ray.

For the same gaze direction (α,β), the image of a point M having a given object proximity is formed between two points S and T which correspond respectively to minimal and maximal focal distances (which would be sagittal and tangential focal distances). The quantity *Prox I* is called image proximity of the point M:

$$ProxI = \frac{1}{2}\left(\frac{1}{JT} + \frac{1}{JS}\right)$$

By analogy with the case of a thin lens, it can therefore be defined, for a given gaze direction and for a given object proximity, i.e. for a point of the object space on the corresponding light ray, an optical power *Pui* as the sum of the image proximity and the object proximity, i.e.

$$Pui = ProxO + ProxI$$

With the same notations, an astigmatism *Ast* is defined for every gaze direction and for a given object proximity as:

$$Ast = \left|\frac{1}{JT} - \frac{1}{JS}\right|$$

This definition corresponds to the astigmatism of a ray beam created by the lens.

Possible definitions of the optical power and the astigmatism of the lens, in usual wearing conditions, can be calculated as explained in the article by B. Bourdoncle et al., entitled "Ray tracing through progressive ophthalmic lenses", 1990 International Lens Design Conference, D. T. Moore ed., Proc. Soc. Photo. Opt. Instrum. Eng.

The prescription in ophthalmic field may comprise, in addition to the power prescription, an astigmatism prescription. Such a prescription is composed of an axis value (in degrees) and a module value (in diopters). The module value represents the difference between the maximal and minimal power in a given direction allowing to correct the visual default of a wearer. Following the convention, the axis represents the orientation of one of the two powers versus a reference axis and following a given rotation direction. TABO convention may be used. In this convention the reference axis is horizontal and the rotation direction is counterclockwise when looking at the wearer. A 45° axis corresponds to an axis orientated obliquely linking, when looking at the wearer, the upper right quadrant to the lower left quadrant. Such an astigmatism prescription is measured for the wearer in far vision. The term 'astigmatism' is used to refer to the couple (module, axis). That term is sometimes used to designate simply the module. The skilled person easily understands what it refers to depending on the context. The skilled person is also aware that the power/astigmatism prescription for a wearer is commonly described with the terms sphere, cylinder and axis.

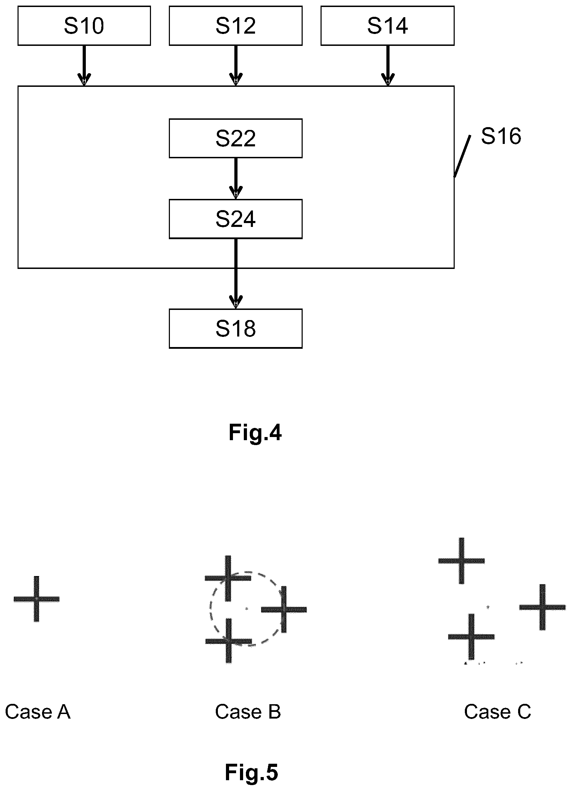

With reference to figure 4, the displaying method comprises at least the following steps:

- a parameter providing step S10,
- a sub-images providing step S12,
- a display device providing step S14,
- an adapting step S16, and
- a displaying step S18.

During the parameter providing step S10, at least one optical parameter relative to the prescription for an eye of the person is provided.

As indicated hereinbefore, the optical parameters may be relative to a dioptric power, an astigmatism and an axis of the eye of the person, for example sphere, cylinder and axis of the eye of the person.

Preferably, at least three optical parameter are provided: sphere, cylinder and axis of the eye of the person.

The optical parameters relative to the prescription of the eye of the person may advantageously be measured beforehand by an eye care practitioner like an optometrist and then provided. The optical parameters relative to the prescription of the eye may be stored on a memory.

A plurality of initial sub-images is provided during the sub-images providing step S12. Each initial sub-image corresponds to at least a part of the image to be displayed on the retina of the eye.

In S14, a plurality of light beams is provided. The light beams are configured to be focused substantially in the plane of a pupil of the eye at a plurality of corresponding different positions also called "focusing points". In the sense of the invention, the light beams are focused substantially in the plane means that the light beams are focused at a maximum distance of 10 mm from the plane of the pupil ensuring the sharp display of images on the retina.

In addition, each light beam is configured to carry an associated sub-image. In other words, each sub-image is carried by an associated light beam focused substantially in the plane of the pupil of the eye.

Thus, each focusing point of the pupil acts as a picoprojector which emits light towards the retina. The focusing of a plurality of light beams in the plane of a pupil of the eye at different positions allows increasing the size of the eye motion box (EMB) and is the basis of pupil extension.

The light beams may be provided using an adapted head-mounted display device. In the sense of the invention, an adapted head-mounted display device is a head-mounted display device configured for displaying an image comprising at least providing a plurality of light beams, each carrying a sub-image and the light beams being adapted to focus substantially in the plane of a pupil of the eye at a plurality of corresponding different positions. For example, US 2016/033771 A1 or WO 2018/091984 A1 both disclose such an adapted head mounted device.

Indeed, the operating principle of the head-mounted display device of US2016/033771 A1 is based on picoprojectors whose emitted light is reflected towards the eye by a holographic mirror. More precisely, this light is focused substantially in the plane of the wearer's pupil (see figures 2A and 2B of US2016/033771 A1). This results in a very small eye motion box. To increase the size of the eye motion box, a holographic mirror focuses the light from the picoprojector at N different positions (see Figures 3A and 3B of US2016/033771 A1, where N=2), each position corresponding to a specific wavelength. These wavelengths are very close to each other, so that the wearer cannot perceive a difference.

The head-mounted display device disclosed in WO2018/091984 A1 is based on a light field display (LFD).

According to an embodiment, the invention relates to a display device adapted to provide a plurality of light beams configured to be focused substantially in the plane of a pupil of the eye at a plurality of corresponding different positions, each light beam being configured to carry an associated sub-image and adapted to execute at least the following steps:

- a parameter providing step S10,
- a sub-images providing step S12,
- a display device providing step S14,
- an adapting step S16, and
- a displaying step S18.

Then for each sub-image, the initial sub-image is adapted based on the at least one provided optical parameter and on the corresponding focused position of the light beam configured to carry the sub-image to form an adapted sub-image during the adapting step S16.

Thus, each sub-image is calculated according to the wearer's needs, i.e. according to the eye's prescription.

Indeed, for a same image to be displayed on the retina of the eye of different persons, these last ones do not perceive this image in the same manner due to their different visual defects of their eyes.

For simplicity's sake, let us consider that the image to be displayed comprises a cross. Figure 5 illustrates the resulting images as seen by persons having different visual defects in the case where three sharp sub-images are displayed and are the same, i.e. a cross localized at the same position in each image. For simplicity's sake let us consider that the three corresponding focusing points are equally distant from the centre of the pupil, and equally distant from each other.

If the person is emmetropic, the person sees a single cross in his/her central vision as illustrated on the left of the figure 5 (Case A). But if the person is myopic or hyperopic, the person sees three distinct crosses, as illustrated at the center of the figure 5 (Case B), whose relative distance is a function of its spherical prescription. Finally, if the person is astigmatic, the person sees also three distinct crosses, as shown on the left of figure 5 (Case C), whose relative horizontal and vertical distances are linked to its cylindrical prescription.

Consequently, each sub-image should be calculated according to the eye's prescription of the person such that the person having visual defects sees a single cross on its retina in the same manner as an emmetropic person.

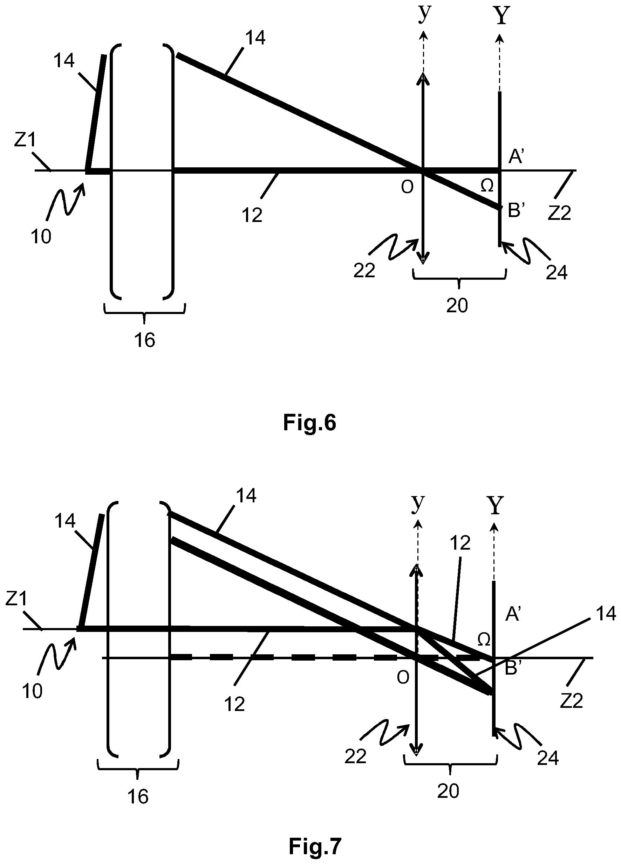

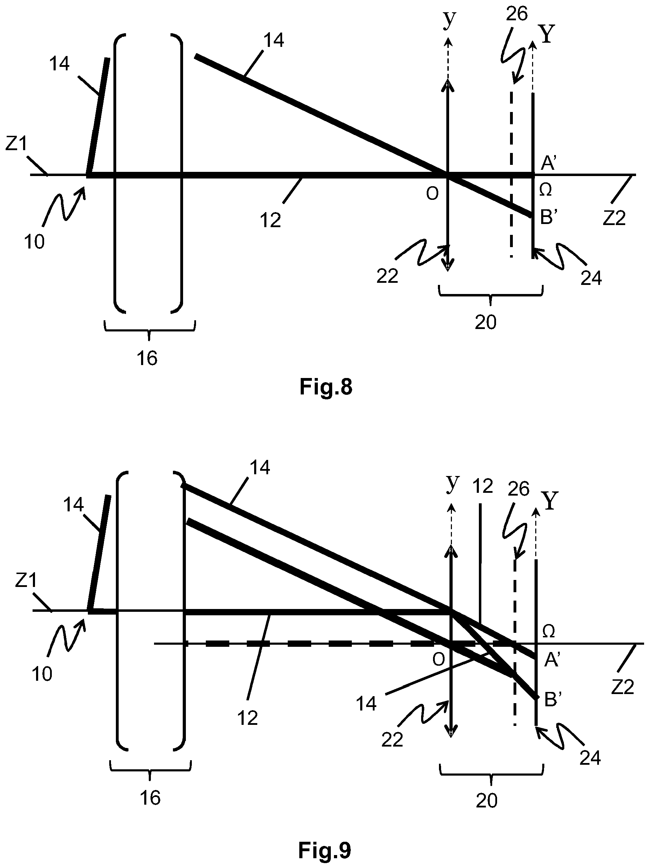

These differences can be explained with regard to figures 6 to 9, illustrating a picoprojector 10 emitting two light rays 12, 14 (only two for simplicity's sake) crossing an optical system 16, represented by the black brackets, towards an eye 20 comprising a pupil 22 and a retina 24. Figures 6 and 7 relate to an emmetropic eye while figures 8 and 9 relate to an ametropic eye, for example a myopic eye.

The optical system 16 is configured to focus the light rays emitted by the picoprojector substantially in the plane of the pupil 22.

On figure 6, when the optical axis Z1 of the picoprojector 10 is coincident with the optical axis Z2 of the eye 20, both light rays 12, 14 focus at the centre of the eye pupil and then form two points A' and B' on the retina 24.

As illustrated on figure 7, when the optical axis Z2 of the eye 20 is shifted in relation to the optical axis Z1 of the picoprojector 10, the position of points A' and B' will not change on the retina 24 because the eye is emmetropic.

However, for a myopic eye as illustrated on figures 8 and 9, light coming from infinity focuses in the dotted plane 26 instead of the retina. Such dotted plane 26 is located before the retina 24 of the eye.

Thus, with reference to figure 8, considering again a single picoprojector 10 focusing at the centre of the eye pupil 22, a sharp image is still displayed on the retina 34.

But, when the optical axis Z2 of the eye is shifted in relation to the optical axis Z1 of the picoprojector 10, the position of points A' and B' are shifted on the retina 24 for the myopic eye as illustrated on figure 9.

As a result, if the person is myopic, light from two picoprojectors displaying the same content will reach the retina at different locations, and the image is no longer sharp on the retina. Instead, two images are shifted and superimposed. And there are as many images as there are focusing points on the eye pupil.

Of course, same observations apply to hyperopic persons and also to astigmatic persons. Preferably, the relative position of each sub-image in the image to be displayed is pre-compensated during the adapting step S16. Adapting the relative position of the sub-image in the image to be displayed comprises:

- a horizontal adapting step S22, and
- a vertical adapting step S24.

During the horizontal adapting step S22, a horizontal angular position of the sub-image carried by the associated light beam is adapted based on the at least one provided optical parameter and on the corresponding focused position of the associated light beam.

Similarly, a vertical angular position of the sub-image carried by the associated light beam is adapted based on the provided optical parameters and on the corresponding focused position of the associated light beam during the vertical adapting step S24.

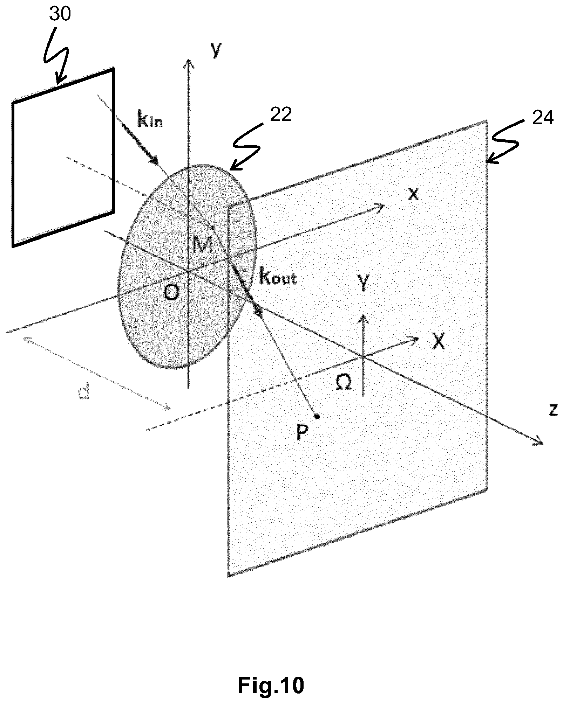

Indeed, with reference to the schematic eye model illustrated on figure 10, a ray directed by wave vector $k_{in}$ coming from the half space in front of the eye and hitting the plane of the pupil 22 (Oxy) at point M is refracted into a wave vector $k_{out}$ by the phase function of the eye when crossing the eye of the pupil and hits the retina 24 at point P.

Point M corresponds to a "focusing point", and the sum of $k_{in}$ vectors correspond to the image content, represented by a square frame 30 in figure 10. The sum of all points P corresponding to all vectors km depict the image generated on the retina.

The coordinates (X,Y) of point P can be determined as follows:

$$\begin{pmatrix} X \\ Y \end{pmatrix} = \frac{d}{c}\begin{pmatrix} a \\ b \end{pmatrix} + \left[1 - \frac{d}{c}\left(\frac{1}{d} + S_{eq}\right)\begin{pmatrix} x \\ y \end{pmatrix}\right] + \frac{d}{c}\frac{C\sin(2\theta)}{2}\begin{pmatrix} y \\ x \end{pmatrix} + \frac{d}{c}\frac{C\cos(2\theta)}{2}\begin{pmatrix} x \\ -y \end{pmatrix} \quad (5)$$

where:

- d is the distance between the plane of the pupil (Oxy) and the retina considered as a plane ($\Omega XY$) for simplicity's sake,
- (*x, y*) are the coordinates of point M in the plane of the pupil,
- (*ka, kb, kc*) are the coordinates of the wave vector **$k_{in}$**, with $k = 2\pi/\lambda$, $\lambda$ is the wavelength and $c = \sqrt{a^2 + b^2}$,
- $S_{eq} = S + C/2$ with S the maximum dioptric power, $C \leq 0$ the astigmatism and $0 \leq \theta \leq 180°$ the axis.

Thus, in the paraxial approximation implying d/c $\approx$ d,, the differences of positions between two points P1 and P2 of the retina, corresponding to two points M1 and M2 lit by corresponding wave vectors **$k_{in,1}$** and **$k_{in,2}$** is expressed by:

$$\begin{pmatrix} X_2 - X_1 \\ Y_2 - Y_1 \end{pmatrix} = d\begin{pmatrix} a_2 - a_1 \\ b_2 - b_1 \end{pmatrix} - dS_{eq}\begin{pmatrix} x_2 - x_1 \\ y_2 - y_1 \end{pmatrix} + d\frac{C\sin(2\theta)}{2}\begin{pmatrix} y_2 - y_1 \\ x_2 - x_1 \end{pmatrix} + d\frac{C\cos(2\theta)}{2}\begin{pmatrix} x_2 - x_1 \\ y_1 - y_2 \end{pmatrix} \quad (6)$$

Thus, when two focusing points M1 and M2 of the pupil are lit by two different rays, the difference of positions of the corresponding points P1 and P2 on the retina depends on the difference of inclination of these rays and on the differences of positions of the focusing points M1 and M2 on the pupil and not on the absolute positions of M1 and M2 on the pupil. Thus, the simultaneous displacement of the focusing points M1 and M2, with the same incident ray directions, only results in a shift of P1 and P2 on the retina.

The focused position in the plane of the pupil of the eye may be determined for each light beam during a preliminary calibration step.

Furthermore, according to a preferred embodiment, the focused positions of the plurality of light beams are advantageously regularly distant from each other in the plane of the pupil of the eye.

During the displaying step S18, each adapted sub-image carried by the associated light beam is displayed on the retina of the person.

The shifting of all the pixels of each sub-image based on the optical parameters of the person's eye allows the sub-images coming from the plurality of focusing points to be superimposed. The sub-images coming from the plurality of focusing point are thus seen as a single sharp image by the person.

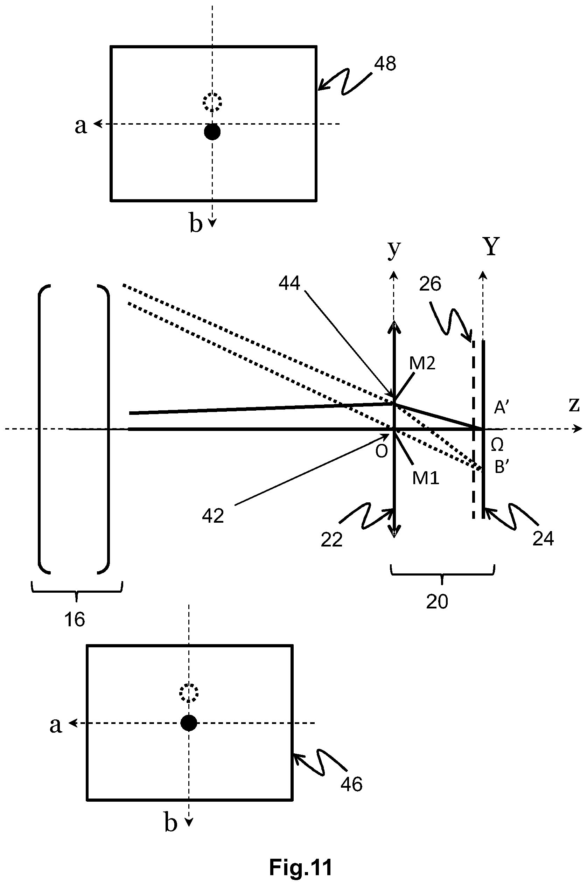

Figure 11 illustrates such method for displaying a sharp image on a retina of an eye of an ametropic person, for example a myopic person. In this example, let us consider that an image content to be displayed (represented by a square frame 30 in figure 10) comprises two discs, one black and one white, aligned vertically.

In addition, only two light beams 42, 44 coming from two picoprojectors, for simplicity's sake, are illustrated focusing substantially in the plane Oxy of the pupil at two corresponding focusing points M1 and M2, aligned on the Oy axis in the example. A first light beam 42 carries an associated sub-image represented by a square frame 46 and a second light beam carries an associated sub-image represented by a square frame 48.

Each sub-image is calculated and adapted according to the prescription of the eye of the person and on the corresponding focused position of the light beam. Thus, the content of the first second image and the second sub-image in their frame are vertically offset from each other allowing the properly superimposition on the retina of the first and second sub-images coming from the focusing points M1 and M2.

The adapted sub-images may be displayed sequentially or simultaneously on the retina of the person.

According to another embodiment, compatible with the previous ones, a wavelength of at least one of the plurality of light beams differs from a wavelength of at least one other of the plurality of light beams. Preferably, the wavelengths are very close to each other so that the person cannot perceive a difference in the image color.

Advantageously, such method allows customizing a head-mounted display device to the user's viewing ability. Indeed, such method allows a pre-compensation of the image to be displayed on the retina of the user based on the prescription of the eye of the user, so as to display a sharp image on the retina of the user.

Consequently, a single head-mounted device could be used by different persons having different prescription, the image to be displayed on the retina of each person being corrected based on a prescription of the eye of the person determined by the same head-mounted device.

As indicated hereinbefore, this method is preferably implemented using a head-mounted device adapted to at least display a plurality of sharp images on the retina of the eye of the person, the plurality of images being carried by a plurality of light beams focused substantially in the plane of the pupil of the eye at different positions. For example the head mounted display devices disclosed in US 2016/033771 A1 or in WO 2018/091984 A1 may be used to implement the method according to the invention.

To this end, a computer program product may be stored in a memory of the head mounted display device, the computer program product comprising one or more stored sequences of instructions that are accessible to a processor of the head mounted display device and which, when executed by the processor, causes the processor to carry out steps of the method according to the invention.

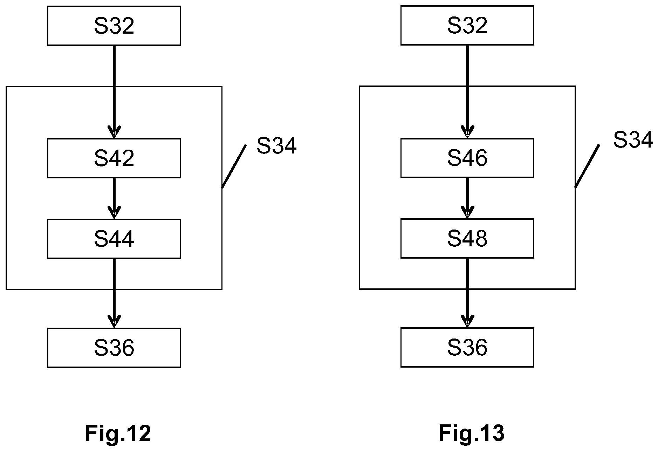

While in the previous embodiment of the displaying method, the optical parameters relative to the prescription of the eye of the person may advantageously be measured beforehand and provided by an eye care practitioner or stored on a memory of the head-mounted device during the parameter providing step S10, the optical parameters relative to the prescription of the eye of the person may advantageously be provided by using same adapted head mounted device to determine them. Indeed, with reference to figure 12, such a method for determining optical parameters of an eye of a person comprises at least the following steps:

- a displaying step S32,
- an adapting step S34, and
- a determining step S36.

During the displaying step S32, three sharp images are displayed on a retina of the eye of the person carried by three light beams. In other words, each sharp image is carried by an associated light beam focused substantially in the plane of a pupil of the eye.

The three light beams are focused substantially in the plane of the pupil of the eye at different positions also called "focusing points".

Thus, each focusing point of the pupil acts as a picoprojector which emits light towards the retina. The focusing of a plurality of light beams in the plane of a pupil of the eye at different positions allows increasing the size of the eye motion box (EMB) and is the basis of pupil extension.

Such displaying step S32 may be implemented using a head-mounted display device as disclosed in US2016/033771 A1 or WO2018/091984 A1.

Each of the three images displayed during the displaying step S2 comprises a target. The target is a spot or a one-dimensional image or a two-dimensional image. The target is for example a symbol or a symbol like a cross as illustrated on figures 5.

Then, a parameter of the target in each image is adapted based on feedback of the person relative to the change of the parameter of the target in the image during the step S34.

With reference to equation (6) and as previously disclosed when two focusing points M1 and M2 of the pupil are lit by two different rays, the difference of positions of the corresponding points P1 and P2 on the retina depends on the difference of inclination of these rays and on the differences of positions of the focusing points M1 and M2 on the pupil and not on the absolute positions of M1 and M2 on the pupil. Thus, the simultaneous displacement of the focusing points M1 and M2, with the same incident ray directions, only results in a shift of P1 and P2 on the retina.

To this end, at each iteration, the parameter of the target in each image is modified and the person provides his/her feedback relative to this change.

The feedback of the person is preferably a vocal feedback, for example provided by answering to questions, for example asking the person if the targets projected on his/her retina are closer or further to each other from an iteration to the following one or other similar questions.

Furthermore, the feedback of the person may be a haptic feedback.

Advantageously, the adapted parameter of the target is preferably the relative position of the target in each image. In this case, the step S34 for adapting a parameter of the target in each image comprises at least a step S42 for adapting a horizontal position of the target of an image and a step S44 for adapting a vertical position of the target of an image.

During the step S42, the horizontal angular position of the target of an image carried by one of the light beams is adapted until the target of the image and the target of another image carried by another of the light beams are seen by the person with the same horizontal position in the displayed images on the retina of the eye.

In the same way, during the step S44, the vertical angular position of the target of an image carried by one of the light beams is adapted until the target of the image and the target of another image carried by another of the light beams are seen by the person with the same vertical position in the displayed images on the retina of the eye.

Thus, during implementation of steps S42 and S44, the values of horizontal and vertical angular position of the target in the images are changed at each iteration to the response of the person when asked if two points of the target projected on his retina are closer or further to each other from an iteration to the following one and this until both targets are seen superimposed by the person, i.e. with the same vertical position and the same horizontal position in the displayed images on the retina of the eye. For visibility's sake, these points can correspond to the center of symbols like crosses for instance.

For example, the crosses can be shifted pixel by pixel in the horizontal and/or in the vertical directions at each iteration.

Advantageously, such method does not require the eye to be totally motionless in relation to the focusing points. The only requirement is that the focusing points are contained in the eye's pupil.

According to a preferred embodiment, the displaying step S32 and the adapting step S34 may be carried out sequentially, i.e. for two of the three light beams and then repeated when the third light beam is added. Indeed, during the displaying step S32, two sharp images are firstly displayed on the retina of the eye of the person carried by two light beams. For both images, a parameter of the target in each image is adapted based on feedback of the person relative to the change of the parameter of the target in the image during the step S34, preferably until the targets of both images are seen by the person with the same position in the displayed images on the retina of the eye. Then, the third sharp image is also displayed on the retina carried by a third light beam and a parameter of the target in the third image is adapted based on feedback of the person relative to the change of the parameter of the target in the image during the step S34, preferably until the targets of the third image and one of the first images are seen by the person with the same position in the displayed images on the retina of the eye.

During the determining step S36, the optical parameter of the person's eye is determined based on the adaption of the parameter of the target in each image.

Indeed, from the changes of the horizontal angular position and the vertical angular position of the target in the images necessary for the person to see only one cross, the optical parameters of the person's eye can be determined.

In other words, from the difference between the initial and final angular positions of the target in the images, in both horizontal and vertical directions, and knowing the positions of the focusing points and a function linking the angular deviation perceived by the person and the real angular deviation of the target in the image, the optical parameters of the person's eye can be determined. The initial position of the target in the images corresponds to the setting of the display device for an emmetropic eye. The final position of the target in the images corresponds to the setting of the display device when adapted to a non-emmetropic person, i.e. when the person sees both targets superimposed.

Indeed, once $(a_1, b_1)$ and $(a_2, b_2)$ are known, the expression (6) leads to two equations for finding the three variables S, C and $\theta$. So, by introducing a third point M3, at least two more equations can be expressed from expression (6), allowing the determination of the three optical parameters S, C and $\theta$.

The position of the focusing points can be beforehand determined during a calibration step.

For example, let us consider that the person is myopic and astigmatic with spherical power error S, cylindrical power C and axis 0°. So, horizontal power error is Sh = S, and vertical power error is Sv = S+C, horizontal and vertical power being the extremum powers, expressed in diopters ($\delta$).

Using a model allowing to link prismatic deviation (in prismatic diopters($\Delta$) and the spherical power error, for example the Prentice's law, the angular deviation perceived by the person between crosses 'i' and 'j' in both horizontal and vertical directions can be expressed:

$$\begin{cases} P_{\Delta,h}(i,j) = d_h(i,j) \times \dfrac{Sh}{10} \\ P_{\Delta,v}(i,j) = d_v(i,j) \times \dfrac{Sv}{10} \end{cases} \qquad (7)$$

where:

- $d_h(i,j)$ is the horizontal distance between focusing point displaying cross 'i' and focusing point displaying cross 'j' (expressed in mm);
- $d_v(i,j)$ is the vertical distance between focusing point displaying cross 'i' and focusing point displaying cross 'j' (expressed in mm);

Thus, if a shift of $n_h(i, j)$ pixels of cross 'j' is necessary to superimposed crosses 'i' and 'j' in the horizontal direction and a shift of $n_v(i, j)$ pixels of cross 'j' is necessary to superimposed crosses 'i' and 'j' in the horizontal direction and in the vertical direction and knowing the angular size of a pixel $dP_\Delta$, which is a build parameter, the angular deviation perceived by the person between crosses 'i' and 'j' in both horizontal and vertical directions can also be expressed:

$$\begin{cases} P_{\Delta,h}(i,j) = n_h(i,j) \times dP_\Delta \\ P_{\Delta,v}(i,j) = n_v(i,j) \times dP_\Delta \end{cases} \qquad (8)$$

From equations (7) and (8), the spherical power error S and the cylindrical power C for the eye of the person are determined.

Of course, such determination can be generalized to prescription having non zero cylinder axis.

Likewise, models other than the Prentice's law can be used to determine the optical parameters of the eye of the person.

While in this embodiment for determining optical parameters relative to power and astigmatism prescriptions of the eye of the person, three sharp images are displayed on the retina of the eye of the person carried by three light beams focused in three different points in the plane of a pupil of the eye, it can be noted that a plurality of sharp images can be displayed on the retina carried by a plurality of associated light beams focused in a plurality of different points in the plane of a pupil of the eye allowing a more accurate determination of the optical parameters relative to its prescriptions.

In addition, the determination of the sphere only, if the cylinder is null requires only the display of two sharp images on the retina of the eye carried by two light beams focused at two different points in the plane of the pupil of the eye.

Of course, while in this embodiment the focusing points are equally distant from the centre of the pupil and equally distant from each other, the method can be generalized to other display device configured to display a plurality of sharp images on the retina of an eye carried by a plurality of associated light beams focused in a plurality of different points in the plane of a pupil of the eye and wherein the focusing points are not equally distant from the center of the eye's pupil and/or not equally distant from each other. In this case, the position of the focusing points should be beforehand determined during a calibration step.

With reference to figure 13, another embodiment of the determining method differs from the previous one in that the adapted parameter of the target is the size of the target in each image instead of the relative position of the target in each image. In this embodiment, the position and the size of the target is the same in each displayed image initially.

In that case, the step S34 for adapting a parameter of the target in each image comprises a step S46 for adapting in a horizontal direction and a step S48 for adapting in a vertical direction.

During the step S46 for adapting in a horizontal direction, the size of the target of an image carried by one of the light beams is adapted in a horizontal direction until the target of the image and the target of another image carried by another of the light beams are seen by the person touching each other in the horizontal direction in the displayed images on the retina of the eye.

In the same way, during the step S48 for adapting in a horizontal direction, the size of the target of an image carried by one of the light beams is adapted in a vertical direction until the target of the image and the target of another image carried by another of the light beams are seen by the person touching each other in the vertical direction in the displayed images on the retina of the eye.

As for the previous method, such determining method is preferably implemented using a head-mounted device adapted to at least display a plurality of sharp images on the retina of the eye of the person, the plurality of images being carried by a plurality of light beams focused substantially in the plane of the pupil of the eye at different positions.

In the same manner, a computer program product may be stored in a memory of the head mounted display device, the computer program product comprising one or more stored sequences of instructions that are accessible to a processor of the head mounted display device and which, when executed by the processor, causes the processor to carry out steps of the method according to the invention and as described hereinbefore.

The method according to the invention allows advantageously customizing a head-mounted display device to the user's viewing ability. Indeed, such method allows a pre-compensation of the image to be displayed on the retina of the user based on the prescription of the eye of the user, so as to display a sharp image on the retina of the user ensuring a use of the head-mounted display device optimized for the user.

Thus, a single head-mounted device could be used by different persons having different prescription, the image to be displayed on the retina of each person being corrected based on a prescription of the eye of the person determined by the same head-mounted device.

The invention has been described above with the aid of embodiments without limitation of the general inventive concept.

Many further modifications and variations will suggest themselves to those skilled in the art upon making reference to the foregoing illustrative embodiments, which are given by way of example only and which are not intended to limit the scope of the invention, that being determined solely by the appended claims.

In the claims, the word "comprising" does not exclude other elements or steps, and the indefinite article "a" or "an" does not exclude a plurality. The mere fact that different features are recited in mutually different dependent claims does not indicate that a combination of these features cannot be advantageously used. Any reference signs in the claims should not be construed as limiting the scope of the invention.

**Claims**

1. Method for displaying a sharp image on a retina (24) of an eye of the person, the person having a prescription for the eye of the person, the method comprising:

   - providing at least one optical parameter relative to the prescription for the eye of the person;
   - providing a plurality of initial sub-images, each initial sub-image corresponding to at least a part of the image to be displayed;
   - providing a plurality of light beams (12, 14) configured to be focused substantially in the plane of a pupil (22) of the eye at a plurality of corresponding different positions, (42, 44) each light beam being configured to carry an associated sub-image;
   - for each sub-image, adapting the sub-image based on the at least one provided optical parameter and on the corresponding focused position of the light beam configured to carry the sub-image to form an adapted sub-image; and
   - displaying each adapted sub-image carried by the associated light beam on the retina (24) of the person.

2. The method according to claim 1, wherein adapting the sub-image comprises adapting the relative position of the sub-image in the image to be displayed.

3. The method according to claim 2, wherein adapting the relative position of the sub-image in the image to be displayed comprises:

   - adapting a horizontal angular position of the sub-image carried by the associated light beam based on the at least one provided optical parameter and on the corresponding focused position of the associated light beam; and
   - adapting a vertical angular position of the sub-image carried by the associated light beam based on the at least one provided optical parameter and on the corresponding focused position of the associated light beam.

4. The method according to any of the preceding claims 1 to 3, wherein the method further comprises for each light beam of the plurality of light beams determining the focused position in the plane of the pupil of the eye.

5. The method according to any of the preceding claims 1 to 4, wherein the focused positions of the plurality of light beams are regularly distant from each other in the plane of the pupil of the eye.

6. The method according to any of the preceding claims 1 to 5, wherein the adapted sub-images are displayed sequentially on the retina of the person.

**EP 4 100 780 B1**

7. The method according to any of the preceding claims 1 to 5, wherein the adapted sub-images are displayed simultaneously on the retina of the person.

8. The method according to any of the preceding claims 1 to 7, wherein a wavelength of at least one of the plurality of light beams differs from a wavelength of at least one other of the plurality of light beams.

9. The method according to the preceding claim 8, wherein the wavelengths of the plurality of light beams are comprises in a narrow band of wavelengths of 50 nm width.

10. The method according to any of the preceding claims 1 to 9, wherein at least three optical parameters relative to the prescription for the eye of the person are provided.

11. The method according to any of the preceding claims 1 to 10, wherein the at least one provided optical parameters of the person's eye is relative to a dioptric power, an astigmatism and an axis of the eye of the person.

12. The method according to any of the preceding claims 1 to 11, wherein providing at least one optical parameter relative to the prescription for the eye of the person comprises:

- displaying at least two sharp images on a retina of the eye of the person, the at least two images comprising a target and being carried by two light beams focused substantially in the plane of a pupil of the eye at at least two different positions;
- adapting a parameter of the target in each image based on feedback of the person relative to the change of the parameter of the target in the image; and
- determining the at least one optical parameter of the person's eye based on the adaption of the parameter of the target in each image.

13. A head-mounted device comprising a plurality of pico-projectors (10) adapted to provide a plurality of light beams (12, 14) configured to be focused substantially in the plane of a pupil (22) of the eye at a plurality of corresponding different positions, (42, 44) each light beam being configured to carry an associated sub-image and a processor adapted to execute at least the steps of the method of claim 1.

14. A computer program product comprising one or more stored sequences of instructions that are accessible to a processor and which, when executed by the processor, causes the processor to carry out at least the steps of claim 1 using the device of claim 13.

15. A computer readable medium carrying one or more sequences of instructions of the computer program product of claim 14.

**Patentansprüche**

1. Verfahren zur Anzeige eines scharfen Bildes auf einer Netzhaut (24) eines Auges der Person, wobei die Person eine Verordnung für das Auge der Person hat, wobei das Verfahren umfasst:

- Bereitstellen von mindestens einem optischen Parameter relativ zu der Verordnung für das Auge der Person;
- Bereitstellen einer Vielzahl von anfänglichen Teilbildern, wobei jedes anfängliche Teilbild mindestens einem Teil des anzuzeigenden Bildes entspricht;
- Bereitstellen einer Vielzahl von Lichtstrahlen (12, 14), die ausgestaltet ist, um im Wesentlichen in der Ebene einer Pupille (22) des Auges an einer Vielzahl von entsprechenden unterschiedlichen Positionen (42, 44) fokussiert zu werden, wobei jeder Lichtstrahl ausgestaltet ist, um ein zugehöriges Teilbild zu tragen;
- für jedes Teilbild Adaptieren des Teilbildes basierend auf dem mindestens einen bereitgestellten optischen Parameter und der entsprechenden fokussierten Position des Lichtstrahls, der ausgestaltet ist, um das Teilbild zu tragen, um ein adaptiertes Teilbild zu bilden; und
- Anzeigen von jedem adaptierten Teilbild, das durch den zugehörigen Lichtstrahl getragen wird, auf der Netzhaut (24) der Person.

2. Verfahren nach Anspruch 1, wobei Adaptieren des Teilbildes Adaptieren der relativen Position des Teilbildes in dem anzuzeigenden Bild umfasst.

3. Verfahren nach Anspruch 2, wobei Adaptieren der relativen Position des Teilbildes in dem Bild, die angezeigt werden soll, umfasst:

- Adaptieren einer horizontalen Winkelposition des Teilbildes, das durch den zugehörigen Lichtstrahl getragen wird, basierend auf dem mindestens einen bereitgestellten optischen Parameter und auf der entsprechenden fokussierten Position des zugehörigen Lichtstrahls; und
- Adaptieren einer vertikalen Winkelposition des Teilbildes, das durch den zugehörigen Lichtstrahl getragen wird, basierend auf dem mindestens einen bereitgestellten optischen Parameter und auf der entsprechenden fokussierten Position des zugehörigen Lichtstrahls.

4. Verfahren nach einem der vorhergehenden Ansprüche 1 bis 3, wobei das Verfahren des Weiteren für jeden Lichtstrahl von der Vielzahl der Lichtstrahlen Bestimmen der fokussierten Position in der Ebene der Pupille des Auges umfasst.

5. Verfahren nach einem der vorhergehenden Ansprüche 1 bis 4, wobei die fokussierten Positionen von der Vielzahl der Lichtstrahlen in der Ebene der Pupille des Auges regelmäßig beabstandet zueinander sind.

6. Verfahren nach einem der vorhergehenden Ansprüche 1 bis 5, wobei die adaptierten Teilbilder sequentiell auf der Netzhaut der Person angezeigt werden.

7. Verfahren nach einem der vorhergehenden Ansprüche 1 bis 5, wobei die adaptierten Teilbilder simultan auf der Netzhaut der Person angezeigt werden.

8. Verfahren nach einem der vorhergehenden Ansprüche 1 bis 7, wobei eine Wellenlänge von mindestens einem von der Vielzahl der Lichtstrahlen sich von einer Wellenlänge von mindestens einem anderen von der Vielzahl der Lichtstrahlen unterscheidet.

9. Verfahren nach dem vorhergehenden Anspruch 8, wobei die Wellenlängen der Vielzahl der Lichtstrahlen in einem schmalen Band von Wellenlängen liegen, das 50 nm Breite hat.

10. Verfahren nach einem der vorhergehenden Ansprüche 1 bis 9, wobei mindestens drei optische Parameter relativ zu der Verordnung für das Auge der Person bereitgestellt werden.

11. Verfahren nach einem der vorhergehenden Ansprüche 1 bis 10, wobei der mindestens eine bereitgestellte optische Parameter des Auges der Person relativ zu einer dioptrischen Brechkraft, einem Astigmatismus und einer Achse des Auges der Person ist.

12. Verfahren nach einem der vorhergehenden Ansprüche 1 bis 11, wobei Bereitstellen des mindestens einen optischen Parameters relativ zu der Verordnung für das Auge der Person umfasst:

- Anzeigen von mindestens zwei scharfen Bildern auf einer Netzhaut des Auges der Person, wobei die mindestens zwei Bilder ein Ziel umfassen und durch zwei Lichtstrahlen getragen werden, die im Wesentlichen in der Ebene einer Pupille des Auges an mindestens zwei unterschiedlichen Positionen fokussiert werden;
- Adaptieren eines Parameters des Ziels in jedem Bild basierend auf Rückmeldung der Person relativ zu der Änderung des Parameters des Ziels in dem Bild; und
- Bestimmen des mindestens einen optischen Parameters des Auges der Person basierend auf der Adaptierung des Parameters des Ziels in jedem Bild.

13. Am Kopf angebrachte ("Head-mounted") Vorrichtung, umfassend eine Vielzahl von Picoprojektoren (10), die adaptiert ist, um eine Vielzahl von Lichtstrahlen (12, 14) bereitzustellen, die ausgestaltet ist, um im Wesentlichen in der Ebene einer Pupille (22) des Auges an einer Vielzahl von entsprechenden unterschiedlichen Positionen (42, 44) fokussiert zu werden, wobei jeder Lichtstrahl ausgestaltet ist, um ein zugehöriges Teilbild zu tragen, und einen Prozessor, der adaptiert ist, um mindestens die Schritte des Verfahrens gemäß Anspruch 1 auszuführen.

14. Computerprogrammprodukt, umfassend eine oder mehrere gespeicherte Sequenzen von Anweisungen, die einem Prozessor zugänglich sind, und die bei Ausführung durch den Prozessor bewirken, dass der Prozessor mindestens die Schritte gemäß Anspruch 1 unter Verwendung der Vorrichtung nach 13 ausführt.

15. Computerlesbares Medium, das eine oder mehrere Sequenzen von Anweisungen des Computerprogrammprodukts gemäß Anspruch 14 trägt.

**Revendications**

1. Procédé permettant d'afficher une image nette sur la rétine (24) d'un œil d'une personne, la personne ayant une ordonnance pour l'œil de la personne, le procédé comprenant :

   - la fourniture d'au moins un paramètre optique relatif à l'ordonnance pour l'œil de la personne ;
   - la fourniture d'une pluralité de sous-images initiales, chaque sous-image initiale correspondant à au moins une partie de l'image à afficher ;
   - la fourniture d'une pluralité de faisceaux lumineux (12, 14) configurés pour être focalisés sensiblement dans le plan de la pupille (22) de l'œil au niveau d'une pluralité de positions différentes correspondantes (42, 44), chaque faisceau lumineux étant configuré pour transporter une sous-image associée ;
   - pour chaque sous-image, l'adaptation de la sous-image en fonction de l'au moins un paramètre optique fourni et de la position focalisée correspondante du faisceau lumineux configuré pour transporter la sous-image afin de former une sous-image adaptée ; et
   - l'affichage de chaque sous-image adaptée transportée par le faisceau lumineux associé sur la rétine (24) de la personne.

2. Procédé selon la revendication 1, dans lequel l'adaptation de la sous-image comprend l'adaptation de la position relative de la sous-image dans l'image à afficher.

3. Procédé selon la revendication 2, dans lequel l'adaptation de la position relative de la sous-image dans l'image à afficher comprend :

   - l'adaptation d'une position angulaire horizontale de la sous-image transportée par le faisceau lumineux associé en fonction de l'au moins un paramètre optique fourni et de la position focalisée correspondante du faisceau lumineux associé ; et
   - l'adaptation d'une position angulaire verticale de la sous-image transportée par le faisceau lumineux associé en fonction de l'au moins un paramètre optique fourni et de la position focalisée correspondante du faisceau lumineux associé.

4. Procédé selon l'une quelconque des revendications 1 à 3 précédentes, dans lequel le procédé comprend en outre, pour chaque faisceau lumineux de la pluralité de faisceaux lumineux, la détermination de la position focalisée dans le plan de la pupille de l'œil.

5. Procédé selon l'une quelconque des revendications 1 à 4 précédentes, dans lequel les positions focalisées de la pluralité de faisceaux lumineux sont régulièrement distantes les unes des autres dans le plan de la pupille de l'œil.

6. Procédé selon l'une quelconque des revendications 1 à 5 précédentes, dans lequel les sous-images adaptées sont affichées séquentiellement sur la rétine de la personne.

7. Procédé selon l'une quelconque des revendications 1 à 5 précédentes, dans lequel les sous-images adaptées sont affichées simultanément sur la rétine de la personne.

8. Procédé selon l'une quelconque des revendications 1 à 7 précédentes, dans lequel une longueur d'onde d'au moins un faisceau de la pluralité de faisceaux lumineux diffère d'une longueur d'onde d'au moins un autre faisceau de la pluralité de faisceaux lumineux.

9. Procédé selon la revendication 8 précédente, dans lequel les longueurs d'onde de la pluralité de faisceaux lumineux sont comprises dans une bande étroite de longueurs d'onde d'une largeur de 50 nm.

10. Procédé selon l'une quelconque des revendications 1 à 9 précédentes, dans lequel au moins trois paramètres optiques relatifs à l'ordonnance pour l'œil de la personne sont fournis.

11. Procédé selon l'une quelconque des revendications 1 à 10 précédentes, dans lequel l'au moins un paramètre optique fourni de l'œil de la personne est relatif à une puissance dioptrique, un astigmatisme et un axe de l'œil de la personne.

**12.** Procédé selon l'une quelconque des revendications 1 à 11 précédentes, dans lequel la fourniture d'au moins un paramètre optique relatif à l'ordonnance pour l'œil de la personne comprend :

- l'affichage d'au moins deux images nettes sur la rétine de l'œil de la personne, les au moins deux images comprenant une cible et étant transportées par deux faisceaux lumineux focalisés sensiblement dans le plan d'une pupille de l'œil au niveau d'au moins deux positions différentes ;
- l'adaptation d'un paramètre de la cible dans chaque image sur la base d'une rétroaction de la personne relative au changement du paramètre de la cible dans l'image ; et
- la détermination de l'au moins un paramètre optique de l'œil de la personne sur la base de l'adaptation du paramètre de la cible dans chaque image.

**13.** Dispositif monté sur la tête comprenant une pluralité de picoprojecteurs (10) conçus pour fournir une pluralité de faisceaux lumineux (12, 14) configurés pour être focalisés sensiblement dans le plan d'une pupille (22) de l'œil au niveau d'une pluralité de positions différentes correspondantes (42, 44), chaque faisceau lumineux étant configuré pour transporter une sous-image associée et un processeur conçu pour exécuter au moins les étapes du procédé selon la revendication 1.

**14.** Produit programme d'ordinateur comprenant une ou plusieurs séquences d'instructions stockées qui sont accessibles à un processeur et qui, lorsqu'elles sont exécutées par le processeur, amènent le processeur à réaliser au moins les étapes de la revendication 1 en utilisant le dispositif de la revendication 13.

**15.** Support lisible par ordinateur transportant une ou plusieurs séquences d'instructions du produit programme d'ordinateur selon la revendication 14.

**Fig.1**

**Fig.2**

**Fig.3**

**Fig.4**

Case A　　　　　　Case B　　　　　　Case C

**Fig.5**

**Fig.6**

**Fig.7**

**Fig.8**

**Fig.9**

**Fig.10**

**Fig.11**

```
        ┌─────────┐                              ┌─────────┐
        │   S32   │                              │   S32   │
        └────┬────┘                              └────┬────┘
     ┌───────┼───────┐                      ┌─────────┼───────┐
     │  ┌────▼────┐   │                      │    ┌────▼────┐   │
     │  │   S42   │   │  ╱ S34               │    │   S46   │   │  ╱ S34
     │  └────┬────┘   │                      │    └────┬────┘   │
     │  ┌────▼────┐   │                      │    ┌────▼────┐   │
     │  │   S44   │   │                      │    │   S48   │   │
     │  └────┬────┘   │                      │    └────┬────┘   │
     └───────┼───────┘                      └─────────┼───────┘
        ┌────▼────┐                              ┌────▼────┐
        │   S36   │                              │   S36   │
        └─────────┘                              └─────────┘
```

**Fig.12**                                   **Fig.13**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 10451895 B2
- EP 3296797 A1
- US 6318859 A

- US 2016033771 A1
- WO 2018091984 A1

### Non-patent literature cited in the description

- **LE GRAND.** Optique Physiologique. Revue d'Optique, 1965, vol. 1

- Ray tracing through progressive ophthalmic lenses. **B. BOURDONCLE et al.** 1990 International Lens Design Conference, D. T. Moore ed., Proc. Soc. Photo. Opt. Instrum. Eng.